# EUROPEAN PATENT SPECIFICATION

(11) **EP 2 322 127 B1**
(45) Date of publication and mention of the grant of the patent: **17.01.2018**
(21) Application number: 09809718.1
(22) Date of filing: 17.07.2009
(51) Int. Cl.: A61F 13/49, A61F 13/15, A61F 13/494, A61F 13/496, A61F 13/511, A61F 13/514

(54) **DISPOSABLE DIAPER**
WEGWERFWINDEL
COUCHE JETABLE

(30) Priority: 01.09.2008 JP 2008224101
(43) Date of publication of application: 18.05.2011
(73) Proprietor: Unicharm Corporation, Ehime 799-0111 (JP)
(72) Inventor: MITSUNO, Satoshi, Kanonji-shi, Kagawa 769-1602 (JP); BABA, Toshimitsu, Kanonji-shi, Kagawa 769-1602 (JP); SAITO, Kyota, Kanonji-shi, Kagawa 769-1602 (JP); HASHIMOTO, Tatsuya, Kanonji-shi, Kagawa 769-1602 (JP); TAKEUCHI, Mariko, Kanonji-shi, Kagawa 769-1602 (JP)
(74) Representative: Peter, Julian
(86) International application number: PCT/JP2009/063001
(87) International publication number: WO 2010/024063

(56) References cited:
- JP-A- 6 054 878
- JP-A- 7 236 650
- JP-A- 8 010 285
- JP-A- 11 004 853
- JP-A- 2001 000 478
- JP-A- 2001 145 666
- US-A1- 2004 122 404
- US-A1- 2004 158 217
- US-B1- 6 264 639
- US-B1- 6 264 641
- US-B1- 6 306 122

## Description

### TECHNICAL FIELD

The present invention relates to disposable pants-type wearing articles such as disposable pants-type diapers or toilet-training pants and particularly to such disposable diapers that liquid-absorbing properties of a liquid-absorbent core would not be disturbed and graphics adapted to be visually recognized from the outside would not be deformed, for example, get wrinkled.

### RELATED ART

Conventionally, pants-type wearing articles are provided in front and rear waist regions with a plurality of waist elastic members extending in a circumferential direction and leg elastic members extending along peripheral edges of the respective leg-openings. For example, PATENT DOCUMENT 1 discloses a disposable diaper comprising a plurality of upper waist elastic members extending along a waist-opening, leg elastic members extending along peripheral edges of respective leg-openings and a plurality of lower waist elastic members extending in the circumferential direction between the upper waist elastic members and the leg elastic members so that stretch properties of these lower waist elastic members could not be expressed in zones of the front and rear waist regions occupied by a liquid-absorbent structure.
PATENT DOCUMENT 1: JP2002-65733 A
Further prior art in this technical field is disclosed in documents US 6,306,122 B1, US 2004/158217 A1, US 6,264,639 B1, US 2004/122404 A1 and US 6,264,641 B1.

### DISCLOSURE OF THE INVENTION

### PROBLEM TO BE SOLVED BY THE INVENTION

In the wearing article according to the invention disclosed by PATENT DOCUMENT 1, stretch properties of the lower waist elastic members are not expressed in the zones of the front and rear waist regions occupied by the liquid-absorbent core. With such an arrangement, the liquid-absorbent core should not get wrinkled under contraction of the lower waist elastic members and, in consequence, a liquid-absorption performance of the liquid-absorbent core should be deteriorated.

Furthermore, if a sheet printed with graphics adapted to be visually recognizable from the outside of the diaper is attached to the zones of the front and rear waist regions occupied by the liquid-absorbent core, the sheet should not get wrinkled under the contractile force of the lower waist elastic members and, in consequence, the graphics should not be undesirably deformed, for example, get wrinkled.

However, when the lower waist elastic members are designed so that the stretch properties of these lower waist elastic members are not expressed at all in the zone occupied by the liquid-absorbent core, segments of the lower waist elastic members defined outside both sides of opposite side edges of the liquid-absorbent core as viewed in a transverse direction should have sufficiently high tensile stress to prevent the diaper from slipping down along the wearer's body during use. With such an arrangement, correspondingly excessive force will be required for the wearer or the helper to broaden the waist-opening by their hands to put the diaper on the wearer's body. This is a problem unsolved from the viewpoint of convenience of handling for the wearer or the helper.

It is an object of the present invention to provide a disposal diaper so improved that the front and rear waist regions as a whole assure an appropriate fit and, in the zones of the front and rear waist regions occupied by the liquid-absorbent core, the diapers include low elastic zones having an appropriate tensile stress of a degree adjusted to eliminate an possibility that the liquid absorption performance of the liquid-absorbent core should be deteriorated and the graphic sheet located in the zones should be deformed.

### MEASURE TO SOLVE THE PROBLEM

According to the present invention, there is provided a disposable diaper having the features according to claim 1.

The present invention further include embodiments as exemplarily described below:
- A graphic sheet printed with graphics adapted to be visually recognized from the outside of the outer sheet is sandwiched between the inelastic zone of the outer sheet and the liquid-absorbent chassis.
- The liquid-absorbent chassis includes side flaps extending outward from opposite side edges of the liquid-absorbent core in the transverse direction and end flaps extending outward from front and rear ends of the liquid-absorbent core in the longitudinal direction and, at the front and rear ends of the liquid-absorbent chassis, the end flaps and the side flaps are attached to the side of the inner sheet facing away from the wearer's skin.
- The liquid-absorbent chassis includes a pair of barrier cuffs formed along the side flaps and front and rear ends of the barrier cuffs are attached to the side of the inner sheet facing away from the wearer's skin.

### EFFECT OF THE INVENTION

In the front and rear waist regions, at least in the rear waist region of the diaper according to the present invention, the zone of the elastic sheet facing the liquid-absorbent core is formed with the low elasticity zone having tensile stress lower than that in other zone of the waist region. With such an unique arrangement, the rear waist region as a whole including the zone occupied by the liquid-absorbent core has an appropriate fit of a degree adjusted to avoid an apprehension that the liquid absorption performance of the liquid-absorbent core should be thereby deteriorated and the graphic sheet located in this zone should be deformed.

### BRIEF DESCRIPTION OF THE DRAWINGS

[FIG. 1] Fig. 1 is a perspective view of a disposable diaper.
[FIG. 2] Fig. 2 is a plan view showing the disposable diaper in a flatly developed state.
[FIG. 3] Fig. 3 is an exploded perspective view of the disposable diaper of Fig. 1.
[FIG. 4] Fig. 4 is an exploded scale-enlarged view of the inner sheet forming a rear waist panel.
[FIG. 5] Fig. 5 is a sectional view taken along line V-V in Fig. 2.
[FIG. 6] Fig. 6 is a plan view is a plan view of the diaper in flatly developed and partially simplified.

### IDENTIFICATION OF REFERENCE NUMERALS USED IN THE DRAWINGS

- 10: disposable diaper
- 11: elastic waist panel
- 12: liquid-absorbent chassis
- 13: front waist region
- 14: rear waist region
- 15: crotch region
- 16: waist-opening
- 17a, 17b: leg-openings
- 18: front waist panel
- 19: rear waist panel
- 20, 21: inner sheets (elastic sheet)
- 22, 23: outer sheets
- 25: inelastic nonwoven fabric
- 26: elastic nonwoven fabric
- 28: liquid-absorbent core
- 30a, 30b: low elasticity zones
- 34, 36: inelastic zones
- 40a, 40b: graphic sheets

### DETAILED DESCRIPTION OF THE PREFERRED EMBODIMENTS

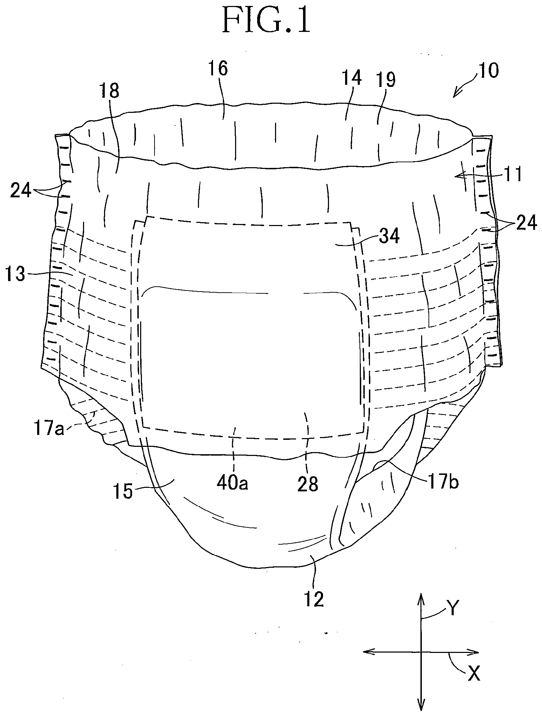

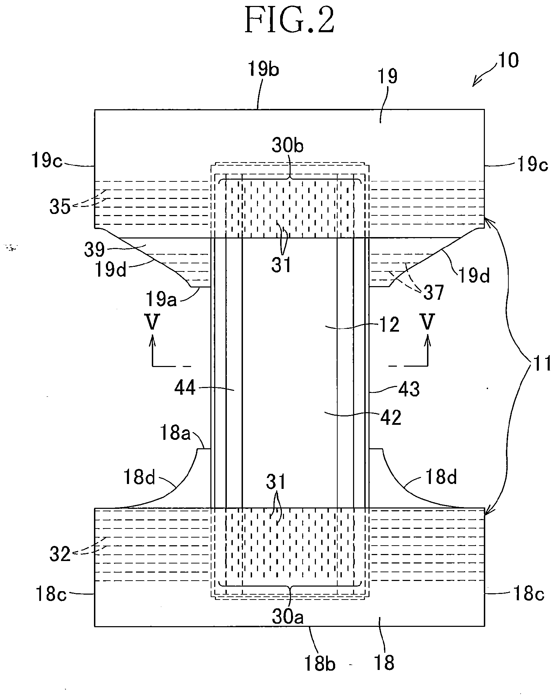

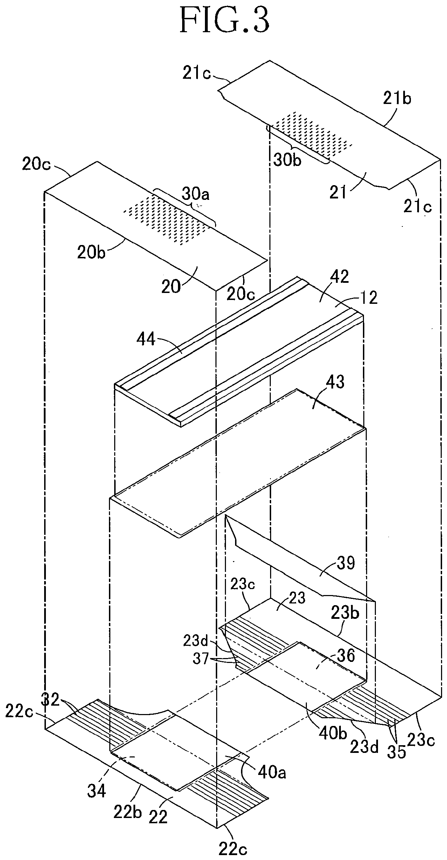

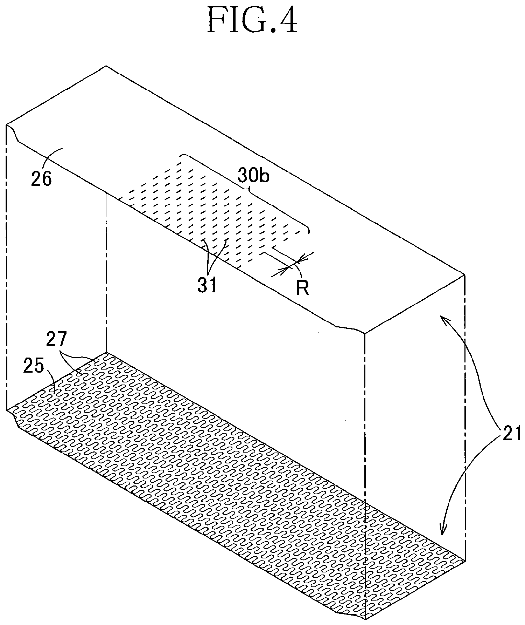

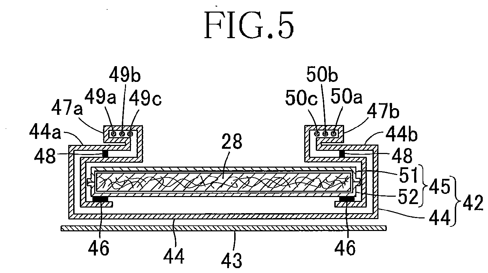

Fig. 1 is a perspective view of a diaper 10, Fig. 2 is a plan view showing a diaper 10 flatly developed in a longitudinal direction Y an a transverse direction X, Fig. 3 is an exploded perspective view of the diaper 10, Fig. 4 is an explodedperspective view of an inner sheet 21 forming a rear waist panel 19 and Fig. 5 is a sectional view taken along line V-V in Fig. 2. The term "elastic" used hereunder means "elastically stretchable". In contrast, the term "inelastic" means "inelastically stretchable".

Referring to Fig. 1, the diaper 10 has the longitudinal direction Y, the transverse direction X and comprises a side facing the wearer' s skin and a side facing away from the wearer' s skin, an annular elastic waist panel 11, a liquid-absorbent chassis 12 attached to the side facing the wearer's skin of the elastic waist panel 11, a front waist panel 13, a rear waist panel 14, a crotch region 15 extending between the front and rear waist regions 13, 14, a waist-opening 16 and a pair of leg-openings 17a, 17b. The waist-opening 16 is defined by the waist panel 11 having an annular shape.

Referring to Figs. 1 and 2, the elastic waist panel 11 comprises a front waist panel 18 defining the front waist region 13 and a rear waist panel 19 defining the rear waist region 14. The front waist panel 18 has a generally trapezoidal shape contoured by an inner end 18a, an outer end 18b, respective outer side edges 18c, 18c and respective inner side edges 18d, 18d obliquely extending inward from the respective outer side edges 18c, 18c following downward concave curves, respectively.

In a state of the diaper 10 formed into a pants-type, the respective outer side edges 18c 19c of the front and rear waist panels 18, 19 are put flat and joined together along side seams 24 arranged intermittently in the longitudinal direction Y by joining means of well known art, for example, various heat welding techniques such as heat embossing or ultrasonic sealing to define the waist-opening and the front and rear waist panels 18, 19 cooperate with the liquid-absorbent chassis 12 to define a pair of leg-openings 17a, 17b.

Referring to Fig. 3, the front and rear waist panels 18, 19 respectively comprise elastic inner sheets 20, 21 lying on the side facing the wearer's skin and having rectangular shapes being relatively long in the transverse direction and elastic outer sheets 22, 23 having generally trapezoidal shapes and defining the respective outer surfaces of the front and rear waist panels 18, 19. The inner sheets 20, 21 and the outer sheets 22, 23 are put flat together so that longitudinally opposite end portions of the liquid-absorbent chassis 12 are respectively sandwiched between the inner and outer sheets 20, 22 and between the inner and outer sheets 21, 23. Outer ends 20b, 21b of the respective inner sheets 20, 21 are permanently bonded to outer ends 22b, 23b of the respective outer sheets 22, 23 with hot melt adhesives (not shown) to form the outer ends 18b, 19b of the front and rear waist panels 18, 19. Side edges 20c, 20c, 21c, 21c of the respective inner sheets 20, 21 and side edges 22c, 22c, 23c, 23c of the respective outer sheets 22, 23 are put flat and permanently bonded together with hot melt adhesives (not shown) to form the outer side edges 18c, 18c, 19c, 19c of , the front and rear waist panels 18, 19.

While the front and rear waist panels 18, 19 respectively comprise the inner and outer sheets 20, 21, 22, 23 according to the present embodiment, it is also possible to form these two panels 18, 19 by the inner sheets 20, 21 only. In this case, the front and rear ends of the liquid-absorbent chassis 12 may be attached to the side of the respective inner sheets 20, 21 facing away from the wearer's skin.

As materials for the inner sheets 20, 21, although not meant to be limited, an elastic laminate fibrous nonwoven fabric may be used and, as materials for the outer sheets 22, 23, although not meant to be limited, a laminate sheet composed of a hydrophobic fibrous nonwoven fabric and a moisture-pervious or a moisture-impervious plastic film may be suitably used.

As specifically shown in Fig. 4, the inner sheet 21 of the rear waist panel 19 is formed of a laminate sheet composed of an inelastic nonwoven fabric 25 and an elastic nonwoven fabric 2 6 bonded under tension to the inelastic nonwoven fabric 25 wherein these nonwoven fabric sheets 25, 26 are bonded to each other with hot melt adhesives 27 applied to the side of the inelastic nonwoven fabric 25 facing the wearer's skin in a wavy pattern. More specifically, the elastic nonwoven fabric 26 is obtained by mechanically stretching a mixed fibrous nonwoven fabric comprising elastomer fibers and inelastic fibers, for example, by a stretching roll. Preferably, the elastic nonwoven fabric 26 stretched to about 1.5 to about 3 times of its initial dimension in the transverse direction X is bonded to the inelastic nonwoven fabric 25. If the stretch ratio is 1.5 or less, the elastic stretchability desired to the effect as will be described later can not be expressed depending on a condition in which the elastic nonwoven fabric 26 has been bonded to the inelastic nonwoven fabric 25 and a quantity of the hot melt adhesives consumed. In contrast, if the stretch ratio is 3 or more, there is a possibility that the mixed fibers might be broken in the course of roll stretching the fixed fiber nonwoven fabric and, in consequence, working might become difficult.

A quantity of the hot melt adhesives 27 used to bond the elastic nonwoven fabric 26 to the inelastic nonwoven fabric 25 is preferably in a range of 1 to 10g/m². If the quantity of the hot melt adhesives 27 is 1g/m² or less, the elastic nonwoven fabric 26 can not be reliably bonded to the inelastic nonwoven fabric , 25 and therefore these nonwoven fabric sheets 25, 26 should be peeled off from each other in the course of making the diaper 10 or during use of the diaper 10. In contrast, if the quantity of the hot melt adhesives 27 is 10g/m² or more, the elasticity of the elastic nonwoven fabric 26 should be inhibited and the desired stretching force could not be expressed. In addition, the hot melt adhesives 27 should leach out to the outer surfaces of the respective nonwoven fabric sheets 25, 26.

While only the inner sheet 21 forming the rear waist panel 19 is shown in Fig. 4, it should be appreciated that the inner sheet 20 forming the front waist panel 18 also has the similar arrangement.

The respective inner sheets 20, 21 arranged in the manner as has been described above are intermittently cut in zones thereof occupied by a liquid-absorbent core 28 to form low elasticity zones 30a, 30b, respectively. Specifically, in vicinities of respective middle zones of the front and rear waist regions 13, 14 occupied by the liquid-absorbent core 28, the elastic nonwoven fabric 26 is intermittently cut by a plurality of slits 31 each extending in the longitudinal direction and arranged in a
staggered pattern in the longitudinal direction Y so that the stretching force of the elastic nonwoven fabric 26 is reduced in the respective slits (i.e., low elasticity zones 30a, 30b). In this way, a relatively low tensile stress of a degree adjusted to be free from adversely affecting the liquid absorption performance of the liquid-absorbent core 28 can be expressed in these low elasticity zones 30a, 30b.

As the method for intermittently cutting the elastic nonwoven fabric 26, various cutting means such as a heat sealing cutter, an ultrasonic sealing cutter, a press cutter or a water jet cutter may be used. While the slits are formed in the staggered pattern according to the present embodiment, the other various cutting patterns may be selectively used (not part of the invention) so far as the initial stretching force of the elastic nonwoven fabric 26 can be reduced to the desired low stretching force. It should be noted here that each of the slits 31 defining the cutting pattern has a length preferably in a range of 3 to 5mm depending on a size of the elastic nonwoven fabric 26. If the length of the individual slit 31 is 3mm or shorter, the surface pressure of the cutter is not sufficiently exerted on the elastic nonwoven fabric 26 and, in consequence, the component fibers of the elastic nonwoven
fabric 26 should be inadequately cut. In contrast, if the length of the individual slit is 5mm or longer, an opening formed by the individual slit 31 will become unacceptably large and the elastic nonwoven fabric 26 should be broken along peripheral edges of the respective openings during use of the diaper 10.

In rows extending in the longitudinal direction Y each comprising a plurality of cuts (slits) 31 each extending in the longitudinal direction Y, a distance R between each pair of the slits 31 which are adjacent in the longitudinal direction Y is preferably in a range of 1 to 7mm. If the distance R is 1mm or shorter, each pair of the adjacent slits 31, 31 may be apt to become continuous with each other and eventually the elastic nonwoven fabric 26 should be broken during use of the diaper 10. In contrast, if the distance R is 7mm or longer, the zone of the elastic nonwoven fabric 26 occupied by the liquid-absorbent core 28 should be subjected to the stretching force of the elastic nonwoven fabric 26 and adversely affect the liquid absorption performance of the liquid-absorbent core 28.

Although not shown, it is also possible to form the inner sheets 20, 21 of, the elastic nonwoven fabric 26 only or of a laminate sheet consisting of an elastic sheet made of nonwoven fabric or elastic plastic and the elastic nonwoven fabric 26. In the latter case, after the inner sheets 20, 21 have been formed, the laminate may be formed with the slits by the heat sealing cutter or the like and thereby not only the elastic nonwoven fabric 26 but also the elastic sheet may be formed with the low elasticity zones.

Referring again to Fig. 3, the outer sheet 22 forming the front waist panel 18 is provided on its side facing the wearer's skin with a plurality of strand-like first waist elastic elements 32 extending in the transverse direction X. More specifically, these first waist elastic elements 32 are located in a middle zone of the front waist region 13 as viewed in the longitudinal direction Y and divided in two groups by the first inelastic zone 34 facing the low elasticity zone 30a so as to be opposed to each other in the transverse direction X. The first waist elastic elements 32 are bonded to the outer sheet 22 with hot melt adhesives (not shown) and covered with the inner sheet 20.

In a similar fashion, the outer sheet 23 forming the rear waist panel 19 is provided on its inner surface with a plurality of strand-like second waist elastic elements 35 extending in the transverse direction X between the outer side edges 19c, 19c. More specifically, these second waist elastic elements 35 are located in a middle zone of the rear waist region 14 as viewed in the longitudinal direction Y and divided in two groups by a second inelastic zone 36 facing the low elasticity zone 30b so as to be opposed to each other in the transverse direction X. In addition to the second waist elastic elements 35, a plurality of strand-like third waist elastic elements 37 extend in the transverse direction X between the oblique inner side edges 23d, 23d and are divided also in two groups by the second inelastic zone 36. The second waist elastic elements 35 are covered with the inner sheet 21 and the third waist elastic elements 37 are covered with an elastic or inelastic auxiliary sheet 39 extending in the transverse direction X between the oblique inner side edges 23d, 23d and having a generally trapezoidal shape.

The term "inelastic zone" used herein includes a zone in which the elastic elements are cut or removed and substantially none of the elastic elements is present and a zone in which contractile properties of the elastic elements is not expressed. The latter zone may be obtained by arranging the respective waist elastic elements 32, 35, 37 so as to extend continuously between the respective outer side edges 22c, 22c; 23c, 23c of the outer sheets 22, 23 and fixing the respective segments of these elastic elements facing the first and second inelastic zone 34, 36 by means of hot melt adhesive or the like so that the contractile properties of these fixed segments can not be substantially expressed.

The term "low elasticity zone" used herein includes a zone having a tensile stress lower than that in the elastic zone provided with the first and second waist elastic elements 32, 35 and the low elasticity zones 30a, 30b of the elastic nonwoven fabric 26 having a tensile stress lower than that in other zones of the elastic nonwoven fabric 26. In other words, it is also possible to form the low elastic zones30a, 30b by attaching elastic elements or elastic sheets having a tensile stress lower than that in the elastic zones provided with the first and second waist elastic elements 32, 35 to the inner sheets 20, 21 formed of inelastic sheets.

It is not essential to form the low elasticity zones 30a, 30b in the front and rear waist regions 13, 14 and these zones may be formed only in the rear waist region 14. In this case, it is unnecessary to form the first inelastic zones 34 in the front waist region 13.

Graphic sheets 40a, 40b formed of a plastic or nonwoven fabric sheet printed with graphics adapted to be visually recognized from the outside are sandwiched between the respective laminates forming the outer sheets 22, 23 in the first inelastic zone 34 and the second inelastic zone 36 defined in the front and rear waist regions 13, 14, respectively. In other words, the graphic sheets 40a, 40b are located in the first and second inelastic zones 34, 36 in which the respective elastic elements 32, 35, 37 are substantially not present or the contractile properties thereof is not expressed. Consequentially, these graphic sheets 40a, 40b should not be deformed, for example, get wrinkled due to contraction of the respective waist elastic elements 32, 35, 37. It should be appreciated that it is also possible to sandwich the graphic sheets 40a, 40b between the outer sheets 22, 23 and the liquid-absorbent chassis 12.

The liquid-absorbent chassis 12 includes a liquid-absorbent structure 42 containing therein the liquid-absorbent core 28 and an outer sheet 43 defining an outer surface of the crotch region 15 is permanently bonded to a lower surface of the liquid-absorbent structure 42 by means of hot melt adhesives (not shown).

The liquid-absorbent chassis 12 additionally includes end flaps extending from front and rear ends of the liquid-absorbent core 28 in the longitudinal direction Y and side flaps extending from opposite side edges of the liquid-absorbent core 28 in the transverse direction X wherein, at the front and rear ends of the liquid-absorbent chassis 12, the end flaps and the side flaps are attached to the sides or the inner sheets 20, 21 facing the wearer's skin.

Referring to Fig. 5, the liquid-absorbent structure 42 includes a liquid-impervious barrier sheet 44 and a liquid-absorbent pad 45 attached to the side of the barrier sheet 44 facing the wearer's skin as if the liquid-absorbent pad 45 is wrapped with the barrier sheet 44.

Referring to Fig. 5, the barrier sheet 44 has opposite side edges 44a, 44b folded in a Z-shape and fixed to the lower surface of opposite side edges of the liquid-absorbent pad 45 with hot melt adhesives 46. The opposite side edges 44a, 44b folded in a Z-shape define a pair of barrier cuffs 47a, 47b spaced from and opposed to each other in the transverse direction X and extending in the longitudinal direction Y.

In the respective barrier cuffs 47a, 47b, the respective inner surfaces of the opposite side edges 44a, 44b of the barrier sheet 44 folded back so as to overlap each other on both sides of the liquid-absorbent pad 45 are partially bonded together with hot melt adhesives 48 to form sleeve-like free edges. As viewed in the diaper 10 flatly developed, these free edges are folded outward and are provided on the inner surfaces thereof with three elastic elements 49a, 49b, 49c; 50a, 50b, 50c, respectively, attached with hot melt adhesives (not shown).

Though not illustrated, the respective barrier cuffs 47a, 47b are permanently bonded to the side of the inner sheets 20, 21 facing away from the wearer's skin with hot melt adhesives (not shown) in the front and rear waist regions 13, 14. With such an arrangement, body waste discharged onto the liquid-absorbent structure 42 should not leak out of the front and rear waist regions 13, 14 even if such body waste flows toward the front and rear waist regions 13, 14.

While three elastic elements 49a, 49b, 49c; 50a, 50b, 50c are attached within free edges of the respective barrier cuffs 47a, 47b according to the present embodiment, at least one elastic element may be attached within each of the free edges so far as, with the diaper 10 put on the wearer's body, the respective elastic members 49, 50 have a tensile stress necessary to put the free edges in close contact with the wearer' s inguinal regions . The barrier cuffs 47a, 47b may be formed not of the barrier sheet 44 but of a separately prepared elastic sheet.

The liquid-absorbent pad 45 comprises a liquid-pervious inner sheet 51, a liquid-impervious outer sheet 52 and the liquid-absorbent core 28 sandwiched between these inner and outer sheets 51, 52.

The liquid-absorbent core 28 is formed of mixture of fluff pulp, superabsorbent polymer (SAP) and, if necessary, heat-sealable staple fibers and preferably wrapped with a liquid-dispersant sheet such as tissue paper for shape retention. To improve the shape retention and the dispersibility, the liquid-absorbent core 28 is compressed in an hourglass-shape and, in consequence, has stiffness higher than the other sheet members. In other words, the liquid-absorbent core 28 is high rigid or semirigid in comparison with the other sheet members.

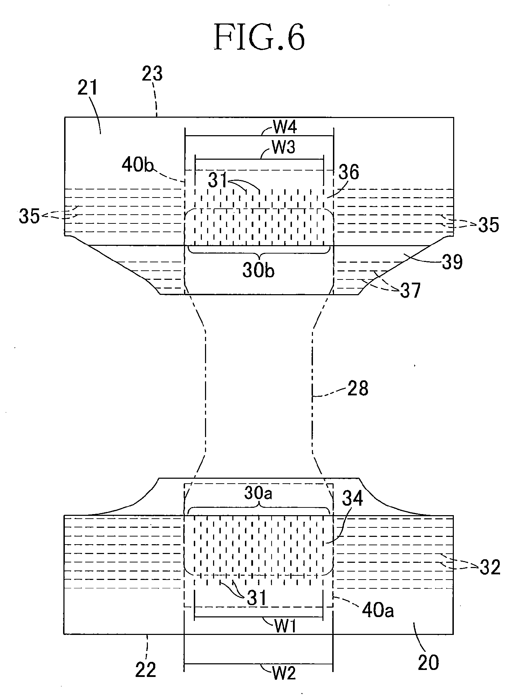

Fig. 6 is a plan view showing the outer sheets 22, 23, the graphic sheets 40a, 40b, the liquid-absorbent core 28 and the inner sheets 20, 21 and all the component elements of the liquid-absorbent chassis 12 except the liquid-absorbent core 28 for convenience of explanation.

As has previously been described, the zones of the front and rear waist regions 13, 14 occupied by the liquid-absorbent core 28 are formed with the first and second inelastic zones 34, 36. With such an arrangement, the liquid absorptive performance should not be adversely affected by contraction of the first through third waist elastic elements 32, 35, 37, the graphic sheets 40a, 40b should be deformed, for example, get wrinkled and/or the graphics should become blur.

Even if the zones of the front and rear waist regions 13, 14 occupied by the liquid-absorbent core 28 are formed with the first and second inelastic zones 34, 36 to solve the problem so that elasticity of the elastic elements or the like is not expressed in these zones at all, the stiffness of liquid-absorbent core 28 higher than that of the other sheet members should prevent the liquid-absorbent core 28 from being put in close contact with the wearer's body so as to leave a gap between the liquid-absorbent core 28 and the wearer's body and eventually bodily fluids should leak out of the diaper 10. Formation of the inelastic zones necessarily deteriorates fit of the diaper 10 as a whole and, in order to assure the desired fit, a tensile stress of the remaining zones, particularly the zones defined on both sides of the inelastic zones must be increased. However, such measure locally increases tensile stress in the inelastic zones and, in consequence, a feeling to wearer the diaper might be deteriorated and compression mark (gather mark) should be left on the wearer's skin.

Such problems may be overcome, according to the present invention, by forming the inner sheets 20, 21 adapted to come in contact with the wearer's skin with the low elasticity zones 30a, 30b and thereby the liquid-absorbent core 28 can be kept in close contact with the wearer's skin and the liquid absorptive performance of the liquid-absorbent core 28 can be assured even in the zones of the front and rear waist regions 13, 14 occupied by the liquid-absorbent core 28. Furthermore, the front and rear waist regions 13, 14 as a whole assure an appropriate fit under the effect of the surface pressure and therefore it is unnecessary to provide the front and rear waist regions with locally high tensile stress which may deteriorate the feeling to wear the diaper 10.

While it may be contemplated to form the outer sheets 22, 23 with the low elasticity zones 30a, 30b, in such case, the elastic nonwoven fabric will be placed closer to the side facing away from the wearer's skin rather than the side of the graphic sheets 40a, 40b. Even if the tensile stress of the elastic nonwoven fabric can be reduced by the low elasticity zones 30a, 30b, the graphic sheets 40a, 40b should be deformed, for example, get wrinkled under contraction of the low elasticity zones 30a, 30b. From the viewpoint of this, it is undesirable to form the outer sheets 22, 23 with the low elasticity zones 30a, 30b.

In order that the present invention can achieve its expected effect, width dimensions W1, W3 of the respective low elasticity zones 30a, 30b in the front and rear waist regions 13, 14 in the transverse direction X are preferably in a range of 0.4 to 0.95 times of width dimension W2, W4 of the liquid-absorbent core 28 facing the low elasticity zones 30a, 30b, respectively, in the transverse direction X. If the width dimensions W1, W3 of the low elasticity zones 30a, 30b in the transverse direction X are 0.4 times or less of the width dimensions W2, W4 of the liquid-absorbent core 28 in the transverse direction X, a portion of the liquid-absorbent core 28 overlapping the zones free from the low elasticity zones could not be kept in close contact with the wearer's skin and a gap should be left between the liquid-absorbent core 28 and the wearer's skin through which bodily fluids should leak out of the diaper 10. This is because the zones of the front and rear waist region 14 occupied by the liquid-absorbent core are formed with the inelastic zones 34, 36 and the stretching force of the first and second waist elastic elements 32, 35 is not expressed in these zones 34, 36. In contrast, if the width dimensions W1, W3 of the low elasticity zones 30a, 30b in the transverse direction X are 0.95 times or more of the width dimensions W2, W4 of the liquid-absorbent core 28 in the transverse direction X, the tensile stress of the low elasticity zones 30a, 30b affects the liquid-absorbent core 28 as a whole and, in consequence, the liquid absorptive performance thereof should be deteriorated.

The diaper 10 according to the present embodiment was evaluated by the method as will be described later and the result of evaluation was indicated by TABLE 1.

### <INVENTIVE EXAMPLE>

The diaper 10 was made in accordance with the illustrated embodiment of the present invention as INVENTIVE EXAMPLE. The low elasticity zones 30a, 30b of the elastic nonwoven fabric 26 forming the inner sheets 20, 21 were formed with the slits 31 in a staggered pattern so that each of the slits 31 has a length of 4mm and the distance R between each pair of the slits 31 being adjacent in the longitudinal direction Y is 4mm.

### <COMPARATIVE EXAMPLE 1>

The diaper of prior art in which the outer sheet is centrally formed with the inelastic zone and an elastic nonwoven fabric is not used as the inner sheet is adopted as COMPARATIVE EXAMPLE 1.

### <COMPARATIVE EXAMPLE 2>

The diaper of prior art in which the outer sheet is centrally formed with the inelastic zone and an elastic nonwoven fabric is used as the inner sheet in a similar fashion to the illustrated embodiment of the present invention (but this elastic nonwoven fabric is not formed with the low elasticity zone) is adopted as COMPARATIVE EXAMPLE 2.

### <Measurement of surface pressure>

Surface pressure (hpa) of the zone in the rear waist region of each diaper occupied by the liquid-absorbent core (i.e., the middle zone of the rear waist region) and the zones defined on laterals of the zone occupied by the liquid-absorbent core and provided with the waist elastic elements (i.e., the laterals of the rear waist region) was measured by the method of well known art.

### <Measurement of width dimension of liquid-absorbent core in used diaper>

The respective liquid-absorbent cores of COMPARATIVE EXAMPLE 1, COMPARATIVE EXAMPLE 2 and INVENTIVE EXAMPLE were dimensioned to have same width (130mm) and this width dimension was measured after the respective diapers have been used for a predetermined time duration.

### <Measurement of displacement of front and rear waist regions in a vertical direction in used diaper>

Displacement of the front and rear waist regions in the vertical direction after the diapers have been used for a predetermined time period with respect to the initial positions immediately after the diapers have been put on the wearer' s body was measured.

**[TABLE 1]**

| | Measurement of surface pressure(hpa) | | Respective dimensions in used diaper (mm) | |
|---|---|---|---|---|
| | Middle of rear waist region (occupied by absorbent core) | Side edges of rear waist region | Width dimension of liquid-absorbent core in transverse direction | Displacement dimension of front and rear waist regions |
| comparative example 1 | 0.2 | 9.3 | 130 | 40 |
| Comparative example 2 | 6.6 | 9.1 | 115 | 40 |
| Inventive example | 3.3 | 6.9 | 128 | 35 |

Referring to TABLE 1, in COMPARATIVE EXAMPLE 1, a central portion of the rear waist region has substantially no surface pressure and high face pressure is concentrated in the side edges of the rear waist region. In COMPARATIVE EXAMPLE 2, substantially no difference of surface pressure can be observed between the central portion and the laterals. In contrast, the surface pressure in the central portion is half or lower of the surface pressure in the laterals in the case of INVENTIVE EXAMPLE.

In COMPARATIVE EXAMPLE 1, elasticity of the waist elastic elements as well as elasticity of the elastic nonwoven fabric is not expressed at all in the central portion of the rear waist region and, as a result, no noticeable change in the width dimension of the liquid-absorbent core is observed. In COMPARATIVE EXAMPLE, the liquid-absorbent core contracts under contractile force of the elastic nonwoven fabric. In contrast, in the case of INVENTIVE EXAMPLE, the liquid-absorbent core slightly contracts but the displacement of the front and rear waist regions is smaller than that observed in COMPARATIVE EXAMPLES 1 and 2.

While the elastic fibrous nonwoven fabric 26 is used as the elastic sheets forming the inner sheets 20, 21, a plurality of strand-like elastic elements may be attached to the inelastic fibrous nonwoven fabric to make it elastic.

As the component elements of the diaper 10 such as the elastic waist panel 11 and the liquid-absorbent chassis 12, various types of material widely used in the related technical field may be selectively used so far as particular materials are not specified as such component elements in this specification. While the front and rear waist regions 13, 14 are formed of the front and rear waist panels 18, 19 according to the present embodiment, it is possible to form the front and rear waist regions 13, 14 by a continuous sheet member integrally forming the outer shape of the diaper as a whole. While the elastic waist panel 11 and the liquid-absorbent chassis 12 are separately prepared according to the illustrated embodiment, it is also possible to sandwich the liquid-absorbent core between a pair of hourglass-shaped elastic waist panels. The present invention is applicable not only to the pants-type disposable diaper but also to the open-type disposable diaper in which the side edges of the front and rear waist regions are not previously joined together.

## Claims

1. A disposable diaper (10) having a longitudinal direction (Y) and a transverse direction (X), comprising a side facing the wearer's skin and a side facing away from the wearer's skin, a front waist region (13), a rear waist region (14), a crotch region (15) extending between said front and rear waist regions (13, 14), a waist-opening (16), a pair of leg-openings (17a, 17b), an elastic front waist panel (18) defining said front waist region (13), an elastic rear waist panel (19) defining said rear waist region (14) and a liquid-absorbent chassis (12) containing a liquid-absorbent core (28), said disposable diaper being **characterized in that**:
said front and rear waist panels (18, 19) respectively include an inner sheet (20, 21) formed of an elastic sheets and lying on said side facing the wearer's skin and an outer sheet (22, 23) having a trapezoidal shape and bonded to at least said side of said inner sheet (20, 21) facing away from the wearer's skin;
front and rear ends portions of said liquid-absorbent chassis (12) are respectively sandwiched between the inner sheet (20, 21) having a rectangular shape and the outer sheet (22, 23) and attached at least to the inner sheet (20, 21);
the elastic sheet defining the inner sheet (20, 21) includes an elastic nonwoven fabric (26) disposed on the side facing the wearer's skin and an inelastic nonwoven fabric (25) disposed on the side facing away from the wearer's skin and the elastic nonwoven fabric (26) is bonded under tension to said inelastic nonwoven fabric (25);
at least in said rear waist region (14) of said front and rear waist regions (13, 14), a portion of said elastic nonwoven fabric (26) corresponding to an end portion of said liquid-absorbent core (28) is partially formed with a low elasticity zone (30a, 30b) having a lower tensile stress than in a remaining region of the elastic nonwoven fabric (26);
the low elasticity zone (30a, 30b) of the inner sheet (20, 21) is formed by cutting the elastic nonwoven fabric (26) by a plurality of slits (31) each extending in the longitudinal direction (Y) and arranged in a staggered pattern;
the outer sheet (22, 23) includes an inelastic nonwoven fabric and a plurality of strand-like elastic elements (32, 35, 37) extending in the transverse direction (X) and attached to the inelastic nonwoven fabric under tension;
a portion of the outer sheet (22, 23) corresponding to both a front and a rear end portion of the liquid-absorbent core (28) is formed with an inelastic zone (34, 36); and
the inelastic zone (34, 36) of the outer sheet (22, 23) is formed by partially cutting or removing the elastic elements (32, 35, 37).

2. The diaper defined by Claim 1, wherein a graphic sheet (40a, 40b) printed with graphics adapted to be visually recognized from the outside of said outer sheet (22, 23) is sandwiched between said inelastic zone of said outer sheet (22, 23) and said liquid-absorbent chassis (12).

3. The diaper defined by Claim 1 or 2, wherein said liquid-absorbent chassis (12) includes side flaps extending outward from opposite side edges of said liquid-absorbent core (28) in said transverse direction (X) and end flaps extending outward from front and rear ends of said liquid-absorbent core (28) in said longitudinal direction (Y); and
at the front and rear end portions of said liquid-absorbent chassis (12), said end flaps and said side flaps are attached to said inner sheet (20, 21) on the side facing away from the wearer's skin.

4. The diaper defined by any one of Claims 1 to 3, wherein said liquid-absorbent chassis (12) includes a pair of barrier cuffs (47a, 47b) formed along said side flaps and front and rear ends of said barrier cuffs (47a, 47b) are attached to said inner sheet (20, 21) on the side facing away from the wearer's skin.

## Patentansprüche

1. Eine Einwegwindel (10) mit einer longitudinalen Richtung (Y) und einer transversalen Richtung (X), die eine der Haut des Trägers zugewandte Seite und eine der Haut des Trägers abgewandte Seite, einen vorderen Taillenbereich (13), einen hinteren Taillenbereich (14), einen Schrittbereich (15), der sich zwischen dem vorderen und hinteren Taillenbereich (13, 14) erstreckt, eine Taillenöffnung (16), ein Paar Beinöffnungen (17a, 17b), ein elastisches vorderes Panel (18), das den vorderen Taillenbereich (13) bestimmt, ein elastisches hinteres Panel (19), das den hinteren Taillenbereich (14) bestimmt, und einen flüssigkeitsabsorbierenden Unterbau (12), der einen flüssigkeitsabsorbierenden Kern enthält, umfasst, **dadurch gekennzeichnet, dass**:
die vorderen und hinteren Panele (18, 19) jeweils eine innere Lage (20, 21), aus einer elastischen Lage gebildet und auf der der Haut des Trägers zugewandten Seite liegend, und eine äußere Lage (22, 23) mit einer Trapezform und mit mindestens der der Haut des Trägers abgewandten Seite der inneren Lage (20, 21) verbunden, umfassen;
vordere und hintere Endbereiche des flüssigkeitsabsorbierenden Chassis (12) jeweils zwischen der inneren Lage (20, 21) mit einer rechteckigen Form und der äußeren Lage (22, 23) liegend und mindestens mit der inneren Lage (20, 21) verbunden sind;
die die innere Lage (20, 21) bestimmende elastische Lage ein elastisches Faservlies (26), das auf der der Haut des Trägers zugewandten Seite angeordnet ist, und ein unelastisches Faservlies (25), das auf der der Haut des Trägers abgewandten Seite angeordnet ist, umfasst und das elastische Faservlies (26) unter Spannung mit dem unelastischen Faservlies (25) verbunden ist;
mindestens im hinteren Taillenbereich (14) der vorderen und hinteren Taillenbereiche (13, 14), ein Bereich des elastischen Faservlieses, einem Endbereich des flüssigkeitsabsorbierenden Kerns (26) entsprechend, teilweise mit einer Zone geringer Elastizität (30a, 30b), mit geringerer Zugspannung als im restlichen Bereich des elastischen Faservlieses (26), ausgebildet ist;
die Zone geringer Elastizität (30a, 30b) der inneren Lage (20, 21) ist durch einschneiden des elastischen Faservlieses (26) mit einer Vielzahl an Schlitzen (31), die sich jeder in longitudinaler Richtung (Y) erstrecken und in einem Zickzack-Muster angeordnet sind, ausgebildet;
die äußere Lage (22, 23) umfasst ein unelastisches Faservlies und eine Vielzahl an strangförmigen, elastischen Elementen (32, 35, 37), die sich in transversaler Richtung (X) erstrecken und unter Spannung an dem unelastischen Faservlies angebracht sind;
ein Bereich der äußeren Lage (22, 23), der sowohl einem vorderen Endbereich, als auch einem hinteren Endbereich des flüssigkeitabsorbierenden Kerns (28) entspricht, mit einer unelastischen Zone (34, 36) ausgebildet ist; und
die unelastische Zone (34 ,36) der äußeren Lage (22, 23) durch teilweises Einschneiden oder Entfernen des elastischen Elements (32, 35, 37).

2. Die Windel gemäß Anspruch 1, wobei eine Grafiklage (40a, 40b) mit Grafiken bedruckt ist, die so angepasst sind, um von außerhalb des flüssigkeitabsorbierenden Kerns (28) optisch erkenntlich zu sein, zwisehen der unelastischen Zone der äußeren Lage (22, 23) und dem flüssigkeitsabsorbierenden Unterboden (12) liegt.

3. Die Windel gemäß Anspruch 1 oder 2, wobei das flüssigkeitsabsorbierende Chassis (12) Seitenlaschen, die sich von den gegenüberliegenden Seitenkanten des flüssigkeitabsorbierenden Kerns (28) nach außen in transversaler Richtung (X) erstrecken, und Endlaschen, die sich von den vorderen und hinteren Enden des flüssigkeitabsorbierenden Kerns (28) nach außen in longitudinaler Richtung (Y) erstrecken, umfasst; und
an den vorderen und hinteren Endbereichen des flüssigkeitsabsorbierenden Chassis (12), die Endlaschen und die Seitenlaschen auf der der Haut des Trägers abgewandten Seite an dem inneren Laken (20, 21) befestigt.

4. Die Windel gemäß einem der Ansprüche 1 bis 3, wobei das flüssigkeitsabsorbierende Chassis (12) ein Paar Sperrbündchen (47a, 47b) umfasst, das entlang der Seitenlaschen ausgebildet ist, und die vorderen und hinteren Enden der Sperrbünde (47a, 47b) auf der der Haut des Trägers abgewandten Seite an der inneren Lage (20, 21) befestigt.

## Revendications

1. Couche jetable (10) ayant une direction longitudinale (Y) et une direction transversale (X), comprenant un côté faisant face à la peau du porteur et un côté opposé à la peau du porteur, une région de taille avant (13), une région de taille arrière (14), une région d'entrejambe (15) s'étendant entre lesdites régions de taille avant et arrière (13, 14), une ouverture de taille (16), une paire d'ouvertures de jambes (17a, 17b), un panneau de taille avant élastique (18) délimitant ladite région de taille avant (13), un panneau de taille arrière élastique (19) délimitant ladite région de taille arrière (14) et un châssis absorbant les liquides (12) contenant un coeur absorbant les liquides (28), ladite couche jetable étant **caractérisée en ce que** :
lesdits panneaux de taille avant et arrière (18, 19) comprennent respectivement une feuille intérieure (20, 21) composée de feuilles élastiques et reposant sur ledit côté faisant face à la peau du porteur et une feuille extérieure (22, 23) ayant une forme trapézoïdale et liée au moins audit côté de ladite feuille intérieure (20, 21) opposé à la peau du porteur ;
des parties finales avant et arrière dudit châssis absorbant les liquides (12) sont respectivement coincées entre la feuille intérieure (20, 21) ayant une forme rectangulaire et la feuille extérieure (22, 23) et attachées au moins à la feuille intérieure (20, 21) ;
la feuille élastique délimitant la feuille intérieure (20, 21) comprend un tissu non-tissé élastique (26) disposé sur le côté faisant face à la peau du porteur et un tissu non-tissé inélastique (25) disposé sur le côté opposé à la peau du porteur et le tissu non-tissé élastique (26) est lié sous tension audit tissu non-tissé inélastique (25) ;
au moins dans ladite région de taille arrière (14) desdites régions de taille avant et arrière (13, 14), une partie dudit tissu non-tissé élastique (26) correspondant à une partie finale dudit coeur absorbant les liquides (28) est partiellement composée d'une zone à faible élasticité (30a, 30b) ayant un effort de traction plus faible que dans une autre région du tissu non-tissé élastique (26) ;
la zone à faible élasticité (30a, 30b) de la feuille intérieure (20, 21) est formée en coupant le tissu non tissé élastique (26) avec une pluralité de fentes (31) s'étendant chacune dans la direction longitudinale (Y) et disposées dans un motif décalé ;
la feuille extérieure (22, 23) comprend un tissu non-tissé inélastique et une pluralité d'éléments élastiques de type fil (32, 35, 37) s'étendant dans la direction transversale (X) et attachés au tissu non-tissé inélastique sous tension ;
une partie de la feuille extérieure (22, 23) correspondant à la fois à une partie finale avant et arrière du coeur absorbant les liquides (28) est composée d'une zone inélastique (34, 36) ; et
la zone inélastique (34, 36) de la feuille extérieure (22, 23) est formée en coupant ou en retirant partiellement les éléments élastiques (32, 35, 37).

2. Couche selon la revendication 1, dans laquelle une feuille graphique (40a, 40b) imprimée avec des graphiques conçus pour être reconnus visuellement depuis l'extérieur de ladite feuille extérieure (22, 23) est coincée entre ladite zone inélastique de ladite feuille extérieure (22, 23) et ledit châssis absorbant les liquides (12).

3. Couche selon la revendication 1 ou 2, dans laquelle ledit châssis absorbant les liquides (12) comprend des rabats latéraux s'étendant vers l'extérieur à partir de bords latéraux opposés dudit coeur absorbant les liquides (28) dans ladite direction transversale (X) et des rabats finaux s'étendant vers l'extérieur à partir d'extrémités avant et arrière dudit coeur absorbant les liquides (28) dans ladite direction longitudinale (Y) ; et
au niveau des parties finales avant et arrière dudit châssis absorbant les liquides (12), lesdits rabats finaux et lesdits rabats latéraux sont attachés à ladite feuille intérieure (20, 21) sur le côté opposé à la peau du porteur.

4. Couche selon l'une quelconque des revendications 1 à 3, dans laquelle ledit châssis absorbant les liquides (12) comprend une paire de revers formant barrière (47a, 47b) formés le long desdits rabats latéraux et des extrémités avant et arrière desdits revers formant barrière (47a, 47b) sont attachées à ladite feuille intérieure (20, 21) sur le côté opposé à la peau du porteur.
